# EUROPÄISCHE PATENTSCHRIFT

(11) **EP 0 689 458 B1**
(45) Veröffentlichungstag und Bekanntmachung des Hinweises auf die Patenterteilung: **25.11.1998**
(21) Anmeldenummer: 94909117.7
(22) Anmeldetag: 07.03.1994
(51) Int. Cl.: A61K 47/22, A61K 9/70, A61K 31/565

(54) **WIRKSTOFFPFLASTER FÜR DIE ABGABE VON ESTRADIOL AN DIE HAUT**
ACTIVE-SUBSTANCE PATCH FOR RELEASING ESTRADIOL TO THE SKIN
EMPLATRE DE MATIERE ACTIVE POUR ADMINISTRATION D'OESTRADIOL PAR LA PEAU

(30) Priorität: 26.03.1993 DE 4309830
(43) Veröffentlichungstag der Anmeldung: 03.01.1996
(73) Patentinhaber: LTS LOHMANN Therapie-Systeme GmbH, 56567 Neuwied (DE)
(72) Erfinder: MURPHY, Teresa, Maria, GB - Gwent NP 44 3PX (GB)
(74) Vertreter: Flaccus, Rolf-Dieter, Dr.
(86) Internationale Anmeldenummer: EP9400670
(87) Internationale Veröffentlichungsnummer: WO9422481

(56) Entgegenhaltungen:
- EP-A- 0 356 382
- DE-A- 2 306 311
- GB-A- 1 385 914

## Beschreibung

Die vorliegende Erfindung betrifft ein Wirkstoffpflaster für die Abgabe von Estradiol an die Haut, welches aus einer Rückschisht und einem damit verbundenen, einen Haftkleber aufweisenden Wirkstoffreservoir besteht, in dem der Wirkstoff zumindest teilweise löslich ist, sowie aus einer den Klebefilm abdeckenden ablösbaren Schutzschicht.

Estradiol wird benötigt zu Linderung der Symptome der Menopause, der Oophorektomie und des primären Hypophysenversagens. Daher besteht zur Linderung monopausaler Symptome wie Hitzewallungen, nervösem Unbehagen und Schlafstörungen ebenso eine bedeutende Notwendigkeit zur Estradiolersatztherapie wie darüberhinaus auch zur Linderung der mit Estradiolmangel einhergehenden Osteoporose (Verlust von knochenmasse) und Atherosklerose.

Bei oraler Gabe von Estradiol, insbesondere von 17-β-Estradiol, ist die Resorption wegen dessen geringer Wasserlöslichkeit nach oraler Verabreichung unbefriedigend. Der rasche Metabolismus von 17-β-Estradiol durch die Leber macht eine hohe Dosis notwendig, die ein häufiges Auftreten von unerwünschten Nebenwirkungen wie z.B. Übelkeit und Thromboembolie zur Folge hat. Es ist daher notwendig, die Mittel und Wege der Estradioltherapie zu verbessern.

Die transdermale Route der parenteralen Verabreichung erlaubt es, zur Vermeidung des first-pass-Metabolismus geringere Dosen von 17-β-Estradiol zu geben. Mit dieser Art der Verabreichung wird somit der Metabolismus relativ großer Mengen von Estradiol vermieden.

Die transdermale Route bietet somit Vorteile gegenüber anderen Routen. Transdermale Systeme zur Verabreichung einer großen Zahl Unterschiedlicher Wirkstoffe oder anderer medikamentöser Mittel sind exemplarisch dargestellt in den US-Patenten: 4,906,169; 5,023,084; 4,818,540 und 4,746,515 sowie in WO 91/05529.
Die dem Stand der Technik entsprechenden transdermalen Systeme sind, was ihre Größe, Dicke und Wirkstoffausnutzung betrifft, weil nur ein geringer Teil der applizierten Dosis therapeutisch genutzt wird, nicht zufriedenstellend. Trotz der Fortentwicklung der Verabreichungsweise besteht daher ein Bedarf an weiter verbesserten Techniken zur Versorgung von Benutzern des erwähnten Medikamentes mit einem anderen konstanten Estradiolspiegel in einer günstigen, d.h. transdermal applizierbaren Dosierweise. Obwohl es grundsätzlich bekannt ist, Estradiol transdermal zu applizieren, ist es aus den vorgenannten Gründen wünschenswert, verbesserte transdermale Prozesse zur Estradiolverabreichung zu entwickeln.

Der Erfindung liegt die Aufgabe zugrunde, ein Wirkstoffpflaster für die Abgabe von Estradiol an die Haut mit einem einen Haftkleber aufweisenden Wirkstoffreservoir, in dem der Wirkstoff zumindest teilweise löslich ist, anzugeben, welches therapeutisch wirksame Estradiolraten liefert, und zwar mit einer erhöhten Bioverfügbarkeit im Vergleich zur oralen oder intramuskulären Gabe des Wirkstoffes. Das Wirkstoffpflaster soll insbesondere infolge seiner vorteilhaften Dosierweise für eine günstige Therapie bei verbesserter Compliance sorgen.

Die Lösung der gestellten Aufgabe gelingt bei einem Wirkstoffpflaster der im Oberbegriff von Anspruch 1 genannten Art mit der Erfindung dadurch, daß das Wirkstoffreservoir eine Polymermatrix ist, der zur Verbesserung der Bioverfügbarkeit des Estradiols ein Penetrationsbeschleuniger der allgemeinen Formel 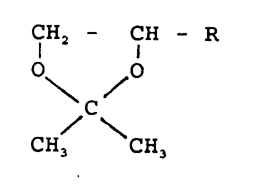 hinzugefügt ist, wobei
R = -CH₂OH oder -CH₂-O-CH₂-CHOH-CH₂OH bedeutet, entsprechend den Verbindungen Monoisopropylidenglycerin (MIPG) oder Monoisopropylidendiglycerin (MIPD).

Mit großem Vorteil wird durch die Hinzufügung des Penetrationsbeschleunigers entsprechend dem Kennzeichen von Anspruch 1 die Bioverfügbarkeit des Wirkstoffs wesentlich verbessert und somit die Applikation einer im Vergleich zu bekannten Systemen geringeren Wirkstoffdosis ermöglicht. Mit Hilfe des erfindungsgemäß zubereiteten Wirkstoffpflasters wird die Bereitstellung der erforderlichen Estradioldosis aus einem transdermalen System verwirklicht.

Eine Ausgestaltung sieht vor, daß das Wirkstoffpflaster Zusätzlich eine oder mehrere weitere Verbindungen enthält, die die Wirkung des Penetrationsverbesserers noch weiter verstärken.
Dabei ist beispielsweise vorgesehen, daß diese penetrationsunterstützenden Verbindungen aus der Gruppe der Polyethylenglykole, Glykole und/oder Pyrrolidone und/oder Polymeren aus Pyrrolidonderivaten ausgewählt sind, beispielsweise Kollidon® 25 (BASF, Homopolymer von N-Vinyl-2-pyrrolidon). Verbindungen der erstgenannten Gruppen sind beispielsweise Propylenglykol (PG), 2-Pyrrolidon (2-P) und Polyethylenglykol 400 (PEG 400).

Eine Ausgestaltung sieht vor, daß das wirkstoffhaltige Reservoir Homo- und/oder Copolymere von Acrylaten und/oder Methacrylaten enthält.

Weiter kann das wirkstoffhaltige Reservoir bis zu 3 Gew.-% Füllstoff enthalten.
Mit Vorteil beträgt die Konzentration des Estradiols im Reservoir 0,7-3,5 Gew.-%.

Dabei kann das Gewichtsverhältnis zwischen Estradiol und Penetrationsbeschleuniger 1:3 bis 1:15 betragen.

Eine Ausgestaltung sieht vor, daß das wirkstoffhaltige Reservoir bis zu 5 Gew.-% eines wasserabsorbierenden Polymers enthält.

Als vorteilhaft hat sich erwiesen, daß das Estradiol das 17-β-Estradiol ist.

Die Erfindung macht Gebrauch von den Prinzipien der transdermalen Wirkstoffabgabe auf den Organismus auf die Haut und ist auf die Verabreichung von Estradiol gerichtet. AuSführungsformen dieser Erfindung verwenden eine Rückschicht, die aus geeignetem Material hergestellt sein kann, welches gegenüber dem Wirkstoff und anderen Bestandteilen der Reservoirschicht undurchlässig ist. Diese Rückschicht erfüllt die erforderliche schützende und stützende Funktion. Geeignete Materialien für die Rückschicht könne sowohl Polyester, Polyvinylchlorid, Polyamid, Polyethylen, Polypropylen und Polyurethan sowie Komposita aus diesen sein. Es können auch metallische Folien wie z.B. aus Aluminium benutzt werden, und zwar entweder alleine oder laminiert mit einer polymeren Substanz.
Eine nach der Erfindung hergestellte, wirkstoffenthaltende Polymermatrix umfaßt den in einem haftklebenden Grundkörper verteilten Wirkstoff, wobei dieser Grundkörper z.B. auf Polyacrylaten oder Polymethacrylaten, Polyurethanen, Silikonen, Polyisobutylenen, Polysiloxanen oder Styren-Isopren-Styren-Copolymeren basieren kann, ebenso wie auf Copolymeren von Ethylen mit Vinylacetat oder Acrylsäurederivaten. Auf diese Weise ist ein enger Kontakt des Wirkstoffes mit der Haut gesichert.

Die genannten Enhancer können einzeln oder auch in Kombination eingesetzt werden. Sie sind verteilt über die gesamte Wirkstoff enthaltende haftklebende Matrix.
Die haftklebende Matrix ist laminiert auf eine geeignete ablösbare Schutzschicht. Eine für solche Laminate geeignete Schutzschicht besteht aus denselben Materialien, wie sie für die Rückschicht beschrieben wurden; sie sind jedoch durch herkömmliche Silikonisierung ablösbar gemacht. Weitere ablösbare Schutzschichten können bestehen aus mit einer Aluminiumfolie laminiertem Polyethylen, wobei die Polyethylenseite, auf die die haftklebende Matrix aufgebracht ist, silikonisiert ist, andere aus Polytetrafluorethylen, vorbehandeltem Papier, Cellophan und ähnlichem.

### Herstellung des Systems

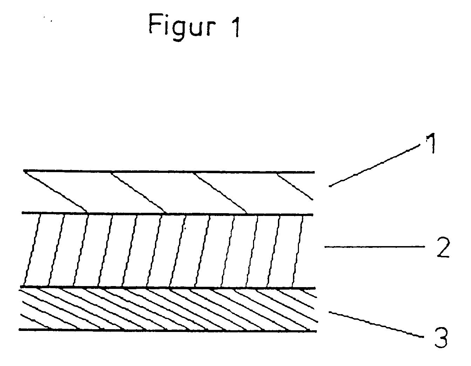

Die bevorzugte Ausgestaltung der vorliegenden Erfindung ist in Figur 1, die einen Querschnitt des erfindungsgemäßen TTS darstellt, veranschaulicht. In dieser Figur 1 bezeichnet (1) die Rückschicht, (2) das den Wirkstoff und Enhancer enthaltende Reservoir und (3) die ablösbare Schutzschicht.
Das den Wirkstoff enthaltende Reservoir kann gebildet werden aus Polymeren von Acrylaten und Methacrylaten. Das den Wirkstoff enthaltende Reservoir kann auch ein wasserabsorbierendes Polymer wie z.B. ein Homopolymer von N-vinyl-2-pyrrolidon enthalten, ebenso auch Füllstoffe, z.B. Aerosil® oder Syloid®.
Der Anteil des eingearbeiteten Füllstoffes kann zwischen 1 und 3 Gew.-% liegen, bevorzugt zwischen 1,5 und 2 Gew.-%.

Der Anteil des eingearbeiteten wasserabsorbierenden Polymers kann bis zu 5 Gew.-% betragen.
Der Wirkstoff und der bzw. die Enhancer sind in verschiedenen Anteilen zwischen 1:3 und 1:15 miteinander kombiniert und sie sind durch Auflösen oder auch feines Verteilen der Wirkstoff-Enhancer-Kombination in einer Polyacrylatlösung eingearbeitet.
Die sich ergebende Mischung wird so lange gerührt, bis eine homogene Dispersion entstanden ist. Die resultierende fließfähige Zubereitung wird flächig verteilt und das Lösungsmittel wird verdampft, so daß man eine lösungsmittelfreie Wirkstoff und Enhancer enthaltende Matrix erhält. Diese Matrix hat die Form eines Films.

Eine typische Verkörperung der Erfindung enthält zwischen 0,7% und 3,5% 17-β-Estradiol (basierend auf den Gewichtsprozent des Films) und zwischen 8 Gew.-% und 28 Gew.-% Enhancer.

Die nachfolgenden Beispiele Zeigen die Realisierung der Erfindung bei einem transdermalen System des Matrixtyps, bei dem die Verabreichung des Wirkstoffs durch das monolithische oder laminierte Matrixsystem diffusionskontrolliert geschieht. Darüberhinaus ist die Erfindung auch anwendbar beim Membrantyp, bei dem die Wirkstoffdiffusion aus dem Reservoir membrankontrolliert erfolgt, wobei das Reservoir mit Flüssigkeit oder halbfestem Stoff gefüllt sein kann.

### Beispiele

Beispiele 1-6: Monolithische Matrixsysteme wurden in Übereinstimmung mit der Erfindung in folgender Weise hergestellt: Der oder die Enhancer wurde(n) intensiv vermischt mit dem Wirkstoff und Siliziumdioxid-Füllstoff (Grace GmbH) und/oder dem wasserabsorbierenden Polymer Kollidon® 25 (BASF). Dazu wurde eine Lösung des Polyacrylat-Haftklebers (Durotak 280-2516, National Starch) gegeben, wobei typische Lösungsmittel für das Polymer niedrigere Alkohole wie Ethanol und Methanol, niedrigere Alkanester wie Ethylacetat und Alkane wie Heptan enthalten. Diese Mischung wurde vollständig zu einer homogenen Masse verrührt. Diese Klebmasse blieb 15 Minuten stehen, bevor sie dann in einer Dicke von 200 µm auf eine silikonisierte Polyesterfolie (Hostaphan RN 100) flächig verteilt und das Lösungsmittel durch Trocknung in einem Ofen bei 50°C entfernt wurde. Die resultierende lösungsmittelfreie Matrix wurde mit einer Polyesterrückschicht (Hostaphan RN 15) laminiert. Aus diesem Laminat wurden Pflaster in einer Größe von 2,54 cm² geschnitten und durch in vitro Hautpermeationsstudien bewertet.

Die silikonisierte Schutzschicht wurde entfernt und die Rückschicht mit der daran klebenden, den Wirkstoff enthaltenden Matrix wurde auf die Stratum-Corneum-Seite von herausgeschnittener nackter Mäusehaut gepreßt. Die Haut wurde dann zusammen mit dem daran befestigten System in eine Franz-Diffusionszelle eingebracht. Das Empfängermedium, 40% Polyethylenglykol in Wasser enthaltend, wurde gerührt und konstant auf einer Temperatur von 37°C gehalten. Muster wurden in vorgegebenen Zeitintervallen entnommen und deren Volumen jeweils sofort ersetzt durch neue schon vorher in einen Gleichgewichtszustand gebrachte Empfängerflüssigkeit. Die Proben wurden mittels HPLC untersucht. Der Wirkstoff-Flux wurde bestimmt aus dem Gefälle der in dem Rezeptormedium vorkommenden kumulierten Wirkstoffmenge im Verhältnis zur Zeit. Die Werte für den Flux in den Beispielen 1-6 sind in Tabelle 1 enthalten. Alle in Tabelle 1 aufgeführten Systeme besaßen akzeptable Flux-Werte, die besten Resultate gab es jedoch beim Einsatz von MIPD allein.

Beispiel 7: Monolithische Matrixsysteme wurden in Übereinstimmung mit der Erfindung hergestellt und bei herausgeschnittener nackter Meerschweinchenhaut zur Anwendung gebracht In vitro-Permeationsexperimente wurden durchgeführt zur Bestimmung der Bioäquivalenz der verschiedenen Systeme. Das auf dem Markt befindliche transdermale System Estraderm-25 TTS wurde dabei als Standard benutzt. Um die Bioäquivalenz der Systeme vergleichen zu können, wurden die Flux-Werte als Prozentsatz der angewandten Dosis ausgedrückt.
Die Daten sind in Tabelle 2 aufgeführt.
Die Ergebnisse zeigen, daß die beste Bioäquivalenz mit einem monolithischen MIPD als Penetrationsbeschleuniger enthaltenden Matrixsystem erzielt wurden. Obwohl dieser Enhancer in gleicher Weise wie Ethanol im Estraderm-TTS agieren kann, d.h. als ein Lösungsmittel für den Wirkstoff, wurde ein überraschend hoher Flux bei diesem monolithischen System erhalten. Auf diese Weise ist es möglich, die Größe des TTS zu reduzieren (auf etwa 4 cm²) und dennoch therapeutisch wirksame Estradioldosen abzugeben (50 µg in 24 Stunden).

Beispiel 8: Wird das MIPD durch MIPG ersetzt, so ergeben sich vergleichbare Resultate.

**Tabelle 1**

| In vitro Flux von Estradiol durch nackte Mäusehaut | | | | | | | | |
|---|---|---|---|---|---|---|---|---|
| Beispiel | (Gew.-% der Lösungsmittelfreien Matrix) | | | | | | | Flux (µg cm⁻²h⁻¹) |
| | Polyacrylat | SiO₂ | PG | Estradiol | MIPD | 2-P | PEG 400 | |
| 1 | 70 | 1,2 | 12 | 1,8 | 15 | / | / | 0,523 ± 0,01 |
| 2 | 70 | 1,2 | 15 | 1,8 | 12 | / | / | 0,523 ± 0,13 |
| 3 | 70 | 1,2 | / | 1,8 | 12 | 15 | / | 0,500 ± 0,03 |
| 4 | 70 | 1,2 | / | 1,8 | 12 | / | 15 | 0,397 ± 0,04 |
| 5 | 70 | 1,2 | 17 | 1,8 | 10 | / | / | 0,523 ± 0,08 |
| 6 | 70 | 2,65 | / | 1,8 | 25,6 | / | / | 0,612 ± 0,09 |

**Tabelle 2**

| In vitro-Flux von Estradiol durch nackte Meerschweinchenhaut | | | | | | | |
|---|---|---|---|---|---|---|---|
| Beispiel | (Gew.-% der lösungsmittelfreien Matrix) | | | | | | Flux (% / h) |
| | Polyacrylat | SiO₂ | PG | Estradiol | MIPD Kollidon-25 | | |
| 7 | 70 | 1,2 | 15 | 1,8 | 12 | / | 12,56 |
| 8 | 70 | 2,65 | / | 1,8 | 25,6 | / | 18,36 |
| 9 | 82,9 | / | / | 2,7 | 10,1 | 4,2 | 21,61 |
| Estraderm.25 TTS | | | | | | | 4,24* |
| (=Stand der Technik) | | | | | | | |

| | | | | | | | |
|---|---|---|---|---|---|---|---|
| * berechnet aus dem in der WO 91/05529 angegebenen Flux | | | | | | | |

## Patentansprüche

1. Wirkstoffpflaster für die Abgabe von Estradiol an die Haut, welches aus einer Rückschicht und einem damit verbundenen, einen Haftkleber aufweisenden Wirkstoffreservoir besteht, in dem der Wirkstoff zumindest teilweise löslich ist, sowie aus einer den Klebefilm abdeckenden ablösbaren Schutzschicht, dadurch gekennzeichnet, daß das Wirkstoffreservoir eine Polymermatrix ist, der zu Verbesserung der Bioverfügbarkeit des Estradiols ein Penetrationsbeschleuniger der allgemeinen Formel 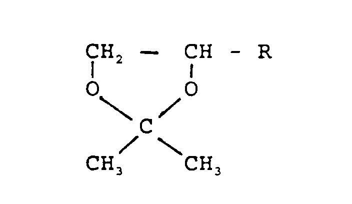 hinzugefügt ist, wobei
R = -CH₂OH oder -CH₂-O-CH₂-CHOH-CH₂OH bedeutet.

2. Wirkstoffpflaster nach Anspruch 1, dadurch gekennzeichnet, daß es zusätzlich eine oder mehrere, die Wirkung des Penetrationsverbesserers verstärkende Verbindungen enthält.

3. Wirkstoffpflaster nach Anspruch 2, dadurch gekennzeichnet, daß die weiteren penetrationsunterstützenden Verbindungen aus der Gruppe der Polyethylenglykole, Glykole und/oder Pyrrolidone und/oder Polymeren aus Pyrrolidonderivaten sind, beispielsweise Kollidon® 25.

4. Wirkstoffpflaster nach Anspruch 1, dadurch gekennzeichnet, daß das wirkstoffhaltige Reservoir Mono- und/oder Copolymere von Acrylaten und/oder Methacrylaten enthält.

5. Wirkstoffpflaster nach Anspruch 1, dadurch gekennzeichnet, daß das wirkstoffhaltige Reservoir bis zu 3 Gew.-% Füllstoff enthält.

6. Wirkstoffpflaster nach Anspruch 1, dadurch gekennzeichnet, daß die Konzentration des Estradiols im Reservoir 0,7 bis 3,5 Gew.-% beträgt.

7. Wirkstoffpflaster nach Anspruch 1, dadurch gekennzeichnet, daß das Gewichtsverhältnis zwischen Estradiol und Penetrationsbeschleuniger 1:3 bis 1:15 beträgt.

8. Wirkstoffpflaster nach Anspruch 1, dadurch gekennzeichnet, daß das wirkstoffhaltige Reservoir bis zu 5 Gew.-% eines wasserabsorbierenden Polymers enthält.

9. Wirkstoffpflaster nach Anspruch 1, dadurch gekennzeichnet, daß das Estradiol das 17-β-Estradiol ist.

## Claims

1. An active substance patch for the release of estradiol to the skin consisting of a backing layer and an active substance reservoir connected thereto which comprises a pressure sensitive adhesive and in which the active substance is at least partially soluble, and a removable protective layer covering the adhesive film, characterized in that the active substance reservoir is a polymer matrix to which, in order to improve the bioavailability of the estradiol, a penetration enhancer of the general formula 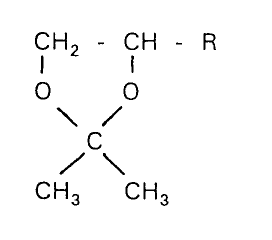 is added, wherein
R = -CH₂OH or -CH₂-O-CH₂-CHOH-CH₂OH.

2. The active substance patch according to claim 1 characterized in that it additionally comprises one or more compounds intensifying the action of the penetration enhancer.

3. The active substance patch according to claim 2 characterized in that the additional penetration-supporting compounds are of the group consisting of polyethylene glycols, glycols and/or pyrrolidones, and/or polymers of pyrrolidone derivatives, for example Kollidon® 25.

4. The active substance patch according to claim 1 characterized in that the active substance-containing reservoir comprises mono and/or copolymers of acrylates and/or methacrylates.

5. The active substance patch according to claim 1 characterized in that the active substance-containing reservoir comprises up to 3%-wt. of a filler.

6. The active substance patch according to claim 1 characterized in that the concentration of estradiol in the reservoir amounts to 0.7 to 3.5%-wt.

7. The active substance patch according to claim 1 characterized in that the weight ratio between estradiol and penetration enhancer amounts to 1:3 to 1:15.

8. The active substance patch according to claim 1 characterized in that the active substance-containing reservoir comprises up to 5%-wt. of a water-absorbing polymer.

9. The active substance patch according to claim 1 characterized in that the estradiol is 17-β-estradiol.

## Revendications

1. Emplâtre pour principe actif pour délivrer de l'oestradiol sur la peau, qui est constitué par une couche dorsale et par un réservoir de principe actif relié à cette dernière, présentant un agent auto-adhésif, dans lequel le principe actif est au moins partiellement soluble, ainsi que par une couche de protection pelliculable recouvrant le film adhésif, caractérisé en ce que le réservoir de principe actif est une matrice polymère à laquelle on a ajouté, pour améliorer la biodisponibilité de l'oestradiol, un accélérateur de la pénétration répondant à la formule générale: 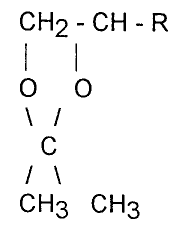 dans laquelle
R représente un groupe -CH₂OH ou un groupe -CH₂-O-CH₂-CHOH-CH₂OH.

2. Emplâtre pour principe actif selon la revendication 1, caractérisé en ce qu'il contient en outre un ou plusieurs composés renforçant l'effet de l'agent d'amélioration de la pénétration.

3. Emplâtre pour principe actif selon la revendication 2, caractérisé en ce que les autres composés favorisant la pénétration sont choisis parmi le groupe comprenant des polyéthylèneglycols, des glycols et/ou des pyrrolidones et/ou des polymères constitués de dérivés de la pyrrolidone, par exemple Kollidon® 25.

4. Emplâtre pour principe actif selon la revendication 1, caractérisé en ce que le réservoir contenant le principe actif contient des mono- et/ou copolymères d'acrylate et/ou de méthacrylate.

5. Emplâtre pour principe actif selon la revendication 1, caractérisé en ce que le réservoir contenant le principe actif contient une matière de charge jusqu'à concurrence de 3% en poids.

6. Emplâtre pour principe actif selon la revendication 1, caractérisé en ce que la concentration de l'oestradiol dans le réservoir s'élève de 0,7 à 3,5% en poids.

7. Emplâtre pour principe actif selon la revendication 1, caractérisé en ce que le rapport pondéral entre l'oestradiol et l'accélérateur de la pénétration s'élève de 1:3 à 1:15.

8. Emplâtre pour principe actif selon la revendication 1, caractérisé en ce que le réservoir contenant le principe actif contient un polymère absorbant l'eau jusqu'à concurrence de 5% en poids.

9. Emplâtre pour principe actif selon la revendication 1, caractérisé en ce que l'oestradiol est le 17-β-oestradiol.
